# EUROPEAN PATENT SPECIFICATION

(11) **EP 2 820 958 B1**
(45) Date of publication and mention of the grant of the patent: **24.10.2018**
(21) Application number: 12813561.3
(22) Date of filing: 09.11.2012
(51) Int. Cl.: A23K 10/37, A23K 50/75, A23K 20/10, A23K 20/158

(54) **COMPOSITION OF LEMON PEEL ESSENTIAL OIL AS A GROWTH PROMOTER IN THE POULTRY INDUSTRY**
COMPOSICIÓN DE ACEITE ESENCIAL DE LIMÓN CÁSCARA COMO PROMOTOR DE CRECIMIENTO EN LA INDUSTRIA AVÍCOLA
COMPOSITION D'HUILE ESSENTIELLE D'ÉCORCE DE CITRON UTILISÉ EN TANT QUE PROMOTEUR DE CROISSANCE DANS L'INDUSTRIE AVICOLE

(30) Priority: 27.02.2012 PE 0002582012
(43) Date of publication of application: 07.01.2015
(73) Proprietor: Universidad Peruana Cayetano Heredia, Lima 31 (PE); San Fernando S.A., Lima 34 (PE); Institut de Recherche pour le Développement, Marseille Cedex 02 (FR)
(72) Inventor: ROJAS DURAN, Rosario Elena, Lima 33 (PE); SHIVA RAMAYONI, Carlos Martín, Lima 41 (PE); KALINOWSKI ECHEGARAY, Juan, Lima 41 (PE); VILLAVICENCIO GALLARDAY, Christian Alejandro, Lima 33 (PE); SAUVAIN, Michel Henri Auguste, F-31100 Toulouse (FR)
(74) Representative: Carvajal y Urquijo, Isabel
(86) International application number: PCT/PE2012/000007
(87) International publication number: WO 2013/129949

(56) References cited:
- FR-A1- 2 853 550
- US-A1- 2003 113 401
- US-A1- 2008 160 000
- US-B2- 6 518 307
- KANG S L ET AL: "FEEDING VALUE OF DRIED CITRUS PEEL IN BROILER DIETS", PROCEEDINGS OF THE SYMPOSIUM OF INTERNATIONAL NETWORK OF FEEDINFORMATION CENTRES, XX, XX, 1 January 1983 (1983-01-01), page 425FF, XP001149813,

## Description

### TECHNICAL FIELD

The invention is referring to a composition based on an extract, which consist on essential oil from Citrus aurantifolia lemon, useful as growth promoter in poultry instead of antibiotics.

This invention is aimed to the extract obtained from lemon, which have a determined composition and used as a supplement in food formulas for poultry, particularly for chickens, so it allows control of microbial gut balance in animals and improves production parameters

The addition of low levels of antibiotics improves growth and food efficiency. The most consistent effect is the intestinal wall thickness, related to inflammation against bacteria and toxins found in intestinal tract. Intestinal infections cause clinical manifestations that affecting animal health, evoking a negative impact in production and affecting economy of poultry productive market.

In response to this problem, growth promoters, commonly corresponding to antibiotics, along with poultry food have been used in a preventive way. A growth promoter is any kind of natural or synthetic compound, with pharmacological activity, that is administered to healthy animals in order to increase weight and improve food efficiency rate.

Antibiotics are used to avoid disease development in poultry, such as diarrheas and other gastrointestinal disorders, which may cause poor food absorption causing animals do not reach an appropriate size and weight for sale. Also, animal mortality decreases using these antibiotics.

A determining factor in growth promoter choice is its local level effect and gastrointestinal tract low absorption in order to get a selective and specific activity in the intestinal lumen to achieve higher potency and effectiveness. Thus, transference of active compounds to animal plasma is avoided, reducing the residual presence in meat and eggs.

The main disadvantage of antibiotics use in animal breeding for human consuming lies in the fact that antibiotics are also used for treatment of several human diseases, resulting dangerous because the risk of antibiotic resistance appearance.

In the document "Assessment of a phytogenic feed additive effect on broiler growth performance, nutrient digestibility and caecal microflora composition", Animal Feed Science and Technology, 2011, 168: 223 - 231, the use of a phytogenic additive obtained from oregano, anise and citric essential oils is evaluated. The difference between such document and the present invention is that the publication does not analyze an only one oil free composition from other plants, instead uses a mixture of three essential oils where is not possible demonstrating the origin of the action of such composition. Such publication does not also corresponds to a define essential oil composition used as an additive.

The document *"*In vitro effect of essential oils from Cinnamomum aromaticum, Citrus limon and Allium sativum on two intestinal flagellates of poultry, Tetratrichomonas gallinarum and Histomonas meleagridis", Parasite, 2003,10(2): 153-7, shows that essential oils would have a preventive action or would serve as treatment against several flagellated parasites in poultry, in organic breeding of poultry or medicaments free poultry. The analysis were done using essential oils from fresh leaves and tested *in vitro* using parasites as *Tetratrichomonas gallinarum* and *Histomonas meleagridis.* The results only showed a possible chemotherapeutic action of active compounds against the named parasites, thus showing only a possible therapeutic action in parasitic diseases, and in contrast to the technical field, the activity is not confirmed in animal. The paper did not also mention use of essential oils as growth factors in healthy animals.

In the paper "The effects of dietary hesperidin supplementation on broiler performance and chicken meat characteristics", Can J Anim Sci, 2011, 91: 275 - 282, an experiment evaluating the effect of using food supplements with bioflavonoid hesperidin (present in fruits from several citric including genus *Citrus*) in growth, build, quality and oxidative stability of broiler chicken breast meat is showed. The use of a control diet and diets with different hesperidin and tocopherol acetate concentrations were compared. The supplements did not improve the final animal weight, weight gain rate, food convers efficiency, inner organs weight or intramuscular fat content. The positive effect of this bioflavonoid was conferred to its antioxidant activity. The present invention does not contain hesperidin and the obtained results in the paper are only points to show the use of phytocompounds to improve poultry meat quality and not its use as growth promoter in poultry industry.

The *in vitro* inhibitory activity of lemon essential oil has been determined in *in vitro* studies against several bacterias such as *Staphylococcus aureus, Bacillus subtilis, Proteus vulgaris, E coli, Klebsiella pneumoniae, Pseudomonas aeruginosa.* (BMC Complement Altern Med, 2006, 6:39). On the other hand, inhibition values on main pathogens involved in food such as *Salmonella* spp and *Escherichia coli* of 98% and for *Staphylococcus aureus* of 100%. (Appl Microbiol, 1970, 19(1):27-31) have been determined.

Such studies only demonstrate *in vitro* activity against specific bacteria. The behavior or *in vivo* effect of lemon essential oil in poultry comes from a series of conditions, antimicrobial activity among them, that is not necessarily identical to that obtained *in vitro,* so these results cannot extrapolated to the use of lemon essential oil as growth promoter in poultry, because *in vitro* inhibition does not guarantee *in vivo* inhibition of pathogenic microorganisms, and even if this could inhibit microorganisms *in vivo,* such results cannot allow to predict that better parameters can be obtained.

### BRIEF DESCRIPTION OF THE INVENTION

The present invention belongs to the field of broilers production technology, specifically it refers to a composition of lemon essential oil used to replace antibiotics as growth promoters in broilers and its use in broiler food production.

The composition corresponds to an essential oil from lemon peel through steam water distillation process.

The chemical composition of lemon essential oil was determined by gas chromatography technique coupled to mass spectrometry in order to obtain an essential oil with a defined chemical composition to ensure product efficacy.

### DETAILED DESCRIPTION OF THE INVENTION

In the present invention, lemon essential oil is used as growth promoter in broilers. The essential oil effectively replaces the use of antibiotics as growth promoters in broilers.

The lemon essential oil is obtained from citric peel, specifically from lemon of *Citrus aurantifolia* species, commonly known as "limon sutil, limon comun, limon criollo or limon peruano". It was decided to investigate this specie on *in vivo* experiments because in our preliminary studies the essential oil showed a good *in vitro* activity against bacterias *Escherichia coli, Campylobacter jejuni, Salmonella typhimurium* and *Enterococcus faecalis.*

The essential oil is obtained from a process that includes a steam distillation step. Its chemical composition was determined by gas chromatography technique coupled to mass spectrometry. This technique allowed to do a quality control to obtained oil with defined chemical composition according to indicated in Table 1, which ensure reproducibility of results on product efficacy.

In general, the procedure to obtain lemon essential oil includes:
Selecting ripe fruits from *Citrus aurantifolia* discarding those in bad condition. Then, cut fruits in half, squeeze to extract juice and put lemon peel together to press. The essential oil is extracted using steam distiller. The distiller is made of stainless steel and includes a 16 L water reservoir, a tripod and a metallic grill, on which between 15 to 25 kg of lemon peel was placed (plant sample is never in direct contact with water). The distiller is hermetically closed and connected to a detachment tube next to a coil condenser that ends in a graduated burette. The next step in the procedure is reservoir water boiling, the water steam passes through the plant sample and sweeping the essential oil; the oil and water steam pass together to the condenser and condensed water is received along with lemon essential oil in a test tube (this tube contains an amount of water that allows visualization of essential oil separation).

The extraction process described above is carried out between 1 and 2 hours, after which the essential oil is separated from aqueous phase. To facilitate the separation, the essential oil is placed in a refrigerator at -4°C. The obtained essential oil is filtered on sodium sulphate anhydrous and the obtained volume is measured.

The lemon essential oil is conserved in amber flasks, to a temperature not above 4 °C, until use.

The yield of extraction, separation and filtration processes is 0.09% (v/w).

According to the global process describe above, a lemon essential oil is obtained with the following composition, as indicated in Table 1.

**Table 1. Composition of lemon peel essential oil.**

| **Compound name** | **% w/v** |
|---|---|
| 2,5-Dihydrotoluene | 0.00 - 0.16 |
| Nonane | 0.00 - 0.06 |
| Tricyclene | 0.00 - 0.04 |
| a-Pinene | 0.40 - 1.70 |
| (1R,4S)-(+)-Canphene | 0.08 - 0.35 |
| Canphene | 0.25 - 0.90 |
| butanoic acid 3-methylbut-2-enil ester | 0.18 - 0.42 |
| β-Pinene | 0.77 - 2.55 |
| β-Miroene | 0.42 - 1.72 |
| 3-Methylen-1,5,5-trimethylcylohexene | 0.00 - 0.09 |
| Pseudolimonene | 0.00 - 0.13 |
| α-Phelandrene | 0.16 - 0.59 |
| Isocineole | 1.66 - 5.31 |
| α-Terpinene | 1.55 - 5.49 |
| o-Cimene | 2.21 - 6.58 |
| D-Limonene | 16.84 - 46.12 |
| β-Phelandrene | 0.23 - 0.65 |
| Eucalyptol | 1.17 - 4.80 |
| tetrahydro-2,2-dimethyl-5-(1-methyl-1-propenyl)-Furan | 0.11 -0.52 |
| β-*cis*-Ocimene | 0.19 - 0.62 |
| (+)-4-Carene | 0.00 - 0.09 |
| y-Terpinene | 5.67 - 19.86 |
| Terpinolene | 4.27 - 12.13 |
| α, *p*-Dimethylstirene | 0.14 - 0.68 |
| β-Linalol | 0.09 - 0.34 |
| 1,3,8-*p*-Mentatriene | 0.00 - 0.08 |
| Myrcenol | 0.00 - 0.17 |
| Fenchol | 0.43 - 1.74 |
| 1-Terpinenol | 0.69 - 2.38 |
| bomyl Chloride | 0.00 - 0.04 |
| β-Terpineol | 0.049 -1.72 |
| α-Terpineol | 0.00 - 0.13 |
| *cis*-*β*-Terpineol | 0.16 - 0.98 |
| Borneol | 0.27 - 1.41 |
| *trans,* 4,5-epoxy-Carane | 0.00 - 0.19 |
| 1-Terpinen-4-ol | 0.47 - 1.73 |
| *p*-Cimen-8-ol | 0.06 - 0.37 |
| *p*-Ment-1-en-8-ol | 4.60 - 14.75 |
| y-Terpineol | 0.64 - 2.48 |
| 1-Decanol | 0.00 - 0.09 |
| *trans*-2-Caren-4-ol | 0.00 - 0.14 |
| (+)-*trans*-Crisantenil acetate | 0.00 - 0.08 |
| *trans*-Pinocarveol | 0.0 0.11 |
| y-Elemene | 0.00 - 0.09 |
| Mircenol | 0.0 1.10 |
| (-)-β-Elemene | 0.00 - 0.08 |
| α-Bergamotene | 0.00 - 0.15 |
| Cariophyllene | 0.16 - 0.77 |
| α-Bergamotene | 0.32 - 1.84 |
| α-Himachalene | 0.00 - 0.07 |
| (Z)-β-Famesene | 0.00 - 0.06 |
| α-Humulene | 0.03 - 0.22 |
| y-Selinene | 0.06 - 0.28 |
| α-Muurolene | 0.00 - 0.08 |
| Isoledene | 0.00 - 0.19 |
| (-)-β-Cadinene | 0.12 - 0.57 |
| α-Farnesene | 0.43 - 1.71 |
| α-Selinene | 0.04 - 0.18 |
| β-Bisabolene | 0.62 - 2.13 |
| α-Longipinene | 0.00 - 0.15 |
| (+)-y-Cadinenoe | 0.00 - 0.09 |
| β-Maaliene | 0.11 - 0.61 |
| Valencene | 0.00 - 0.07 |
| Eudesma-3,7(11 )-diene | 0.00 - 0.21 |
| Cariofilenyl alcohol | 0.00 - 0.09 |
| y-Eudesmol | 0.00 - 0.10 |

Through several experiments performed on thousands of chickens, it is demonstrated that citric essential oil, especially from lemon, has the same effects that antibiotics commonly used in poultry industry, favoring an improved food conversion (parameter that indicates the quantity of food in kg necessary to produce a kg of alive chicken) and lower mortality between chickens.

As a consequence, the use of this kind of essential oil allows the breeding of poultry feed in a natural way, avoiding the use of antibiotics.

Besides, the use of lemon essential oil not only effectively replaces antibiotics as growth promoter, but also improves general health condition of animals, and allows obtaining chickens free of antibiotics as growth promoters.

Based on *in vitro* studies of lemon essential oils and the inhibition produced on several microorganisms, its *in vivo* activity was evaluated on broilers considering the following parameters: gain of weight; food consume; food conversion and mortality.

The experiments were carried out based on the application of 3 different treatments, performing at least 4 repetitions per treatment.

The treatments include a Control group (-): without promoter or extract; a Control group (+): with growth promoter, an antibiotic to be more specific; and a group with a diet supplemented of essential oils from lemon peel.

The experimental animals correspond to male chickens COBB 500, with a breed density of 10.5 animals/m². Also, the infrastructure conditions and barn equipment were evaluated, ensuring these were in optimal conditions and equally assigned between treatments, to minimize its effect on productive results.

### EXAMPLES

### EXAMPLE 1 - Obtaining of essential oil from lemon peel

Ripe fruits from *Citrus aurantifolia* were selected discarding those in bad condition. Then, fruits were cut in half, squeeze to extract juice and lemon peel put together to obtain groups of 20 kg each. The essential oil was extracted using a steam distiller. This distiller is made of stainless steel and includes a 16 L water reservoir, a tripod and a metallic grill, on which 20 kg of lemon peel were placed (plant sample was never in direct contact with water). The distiller is hermetically closed and connected to a detachment tube next to a coil condenser that ends in a graduated burette. Then, the next procedure was water reservoir boil, the water steam generated passes through plant sample and swept the essential oil; the oil and water steam pass together to the condenser and condensed water is received along with lemon essential oil in a test tube (this tube contains an amount of water that allows visualization of essential oil separation).

After 1.5 hours the distillation process was interrupted, then separating essential oil from aqueous phase. To make easier the separation, the essential oil was placed in a refrigerator at -4 °C. The obtained essential oil was filtered on sodium sulphate anhydrous and the volume was measured. The lemon essential oil was conserved in amber flasks, to a temperature not above of 4°C, until use.

From 692 kg of ripe and fresh fruit peels of *Citrus aurantifolia,* 620 ml of essential oil was obtained with a yield of 0.09% v/w.

### EXAMPLE 2- Conditions

Male chicken COBB 500 were evaluated at breed density was 10.5 animals/m², it means, 42 chickens per corral. Twelve corrals with a dimension of 2 x 2 m were used.

The 1 -day animals were placed in corrals with cement floor, rice husk as bed material. The room kept a temperature of 32 - 33°C for chicken reception, for which breeders were switch on at least 1 hour before entry.

The feeders and troughs contained food and water, respectively, was putted before entry of animals, so they could find food and water when they entered to corrals.

At the entry, BB chickens were weighted in groups of 42 per corral, each repetition shall has similar weights so treatments began under same weight conditions.

Weight and food consume measure were performed weekly. The weight at 6th week was individual to evaluate variation between treatments.

During experimental procedure, the recommended health program was applied by the sanitary area.

A diary mortality record was made indicating possible causes after necropsy of all death chickens.

Powder food and water without restriction were administered. Diary pre-weighted food was delivered and remaining food was recorded at the end of the week for measurement of food consume.

The finisher food (40-42 days), was the same for all treatments, because of the fact that growth promoter are normally used until finisher food (34 days), after that it was retired and food was the same for all.

### Assay

The experiments were carried out based on the application of 3 different treatments, performing 4 repetitions per treatment. Treatments were as follows:
- Diet with lemon essential oil 1 kg per metric ton of food (from now on referred as: Kg/TM) (Group 1);
- Control (-): without promoter, without extract (Group 2); and
- Control (+): control Group, with bacitracin methylen disalicilate (BMD) as growth promoter 1 Kg/TM and colistine sulphate 8% 0.25 Kg/TM (Group 3)

According to assay conditions here described, the results obtained are showed in Table 2.

**Table 2. Effects on evaluation parameters according to the treatment.**

| **Experimental Group** | **Weight** (g/chicken) | **Food Consumption** (g/chicken) | **Food Conversion** | **Mortality** (%) |
|---|---|---|---|---|
| Group 1 (with lemon essential oil) | 2552 | 4257 | 1.66 | 1.8 |
| Group 2 (control -) | 2413 | 4277 | 1.79 | 5.4 |
| Group 3 (control +) | 2511 | 4225 | 1.68 | 2.4 |

From Table 2, a better productivity is observed in fed chickens with essential oil versus Group 2 of treatment. Additionally, chicken with essential oil gained better body weight in comparison to animals with (+) and (-) control treatment; at the same time, chickens from Group 1 consumed a similar quantity of food in relation to control treatments, it means, the quantity of consumed food did no decrease, thus ensuring an adequate growth of chickens.

Finally, bearing in mind that Food Conversion is a relation between quantity of consumed food and animal weight, it was observed that in fed chicken with diet containing essential oil this parameter was not significantly affected in comparison to control (+), it means that lemon essential oil is an effective replacement for growth promoter. Now, related to chicken mortality, this parameter is still better for Group 1, it means, less fed chickens with lemon essential oil died which results in batches with higher productivity.

## Claims

1. Essential oil composition from lemon peel useful as growth promoter in poultry,
| | |
|---|---|
| y -Terpineol | 0.64 - 2.48 |
| Cariophyllene | 0.16 - 0.77 |
| α-Bergamotene | 0.32 - 1.84 |
| α-Humulene | 0.03 - 0.22 |
| y-Selinene | 0.06 - 0.28 |
| (-)-β-Cadinene | 0.12 - 0.57 |
| α-Farnesene | 0.43 - 1.71 |
| α-Selinene | 0.04 - 0.18 |
| β-Bisabolene | 0.62 - 2.13 |
| β - Maaliene | 0.11 - 0.61 |

2. Essential oil composition from lemon peel according to claim 1, characterized because it is also constituted for the following compounds:
| **Compound name** | **% w/v** |
|---|---|
| 2,5-Dihydrotoluene | 0.00 - 0.16 |
| Nonane | 0.00 - 0.06 |
| Tricyclene | 0.00 - 0.04 |
| 3-Methyleno-1,5,5-trimethylcyclohexene | 0.00 - 0.09 |
| Pseudolimonene | 0.00 - 0.13 |
| (+)-4-Carene | 0.00 - 0.09 |
| 1,3,8-p-Mentatriene | 0.00 - 0.08 |
| Mircenol | 0.00 - 0.17 |
| Bomyl chloride | 0.00 - 0.04 |
| α-Terpineol | 0.00 - 0.13 |
| trans, 4,5-epoxy-Carane | 0.00 - 0.19 |
| 1-Decanol | 0.00 - 0.09 |
| trans-2-Caren-4-ol | 0.00 - 0.14 |
| (+)-trans-Crisantenil acetate | 0.00 - 0.08 |
| trans-Pinocarveol | 0.00 - 0.11 |
| y-Elemene | 0.00 - 0.09 |
| Mircenol | 0.00 - 1.10 |
| (-)-β-Elemene | 0.00 - 0.08 |
| α-Bergamotene | 0.00 - 0.15 |
| α-Himachalene | 0.00 - 0.07 |
| (Z)-β-Farnesene | 0.00 - 0.06 |
| α-Muurolene | 0.00 - 0.08 |
| Isoledene | 0.00 - 0.19 |
| α-Longipinene | 0.00 - 0.15 |
| (+)-y-Cadinene | 0.00 - 0.09 |
| Valencene | 0.00 - 0.07 |
| Eudesma-3,7(11)-diene | 0.00 - 0.21 |
| Cariofilenyl alcohol | 0.00 - 0.09 |
| y-Eudesmol | 0.00 - 0.10 |

3. Poultry food supplement characterized because it is constituted by 1 kg of essential oil according to claims 1 and 2, per metric ton of food.

## Patentansprüche

1. Ätherische Ölzusammensetzung aus Zitronenschale von Citrus aurantifolia, verwendbar als Wachstumsförderer bei Geflügel.
| | |
|---|---|
| y-Terpineol | 0.64 - 2.48 |
| Caryophyllen | 0.16 - 0.77 |
| α-Bergamoten | 0.32 - 1.84 |
| α-Humulen | 0.03 - 0.22 |
| y-Selinen | 0.06 - 0.28 |
| (-)-β-Cadinen | 0.12 - 0.57 |
| α-Farnesen | 0.43 - 1.71 |
| α-Selinen | 0.04 - 0.18 |
| β-Bisabolen | 0.62 - 2.13 |
| β-Maalien | 0.11 - 0.61 |

2. Ätherische Ölzusammensetzung aus Zitronenschale nach Anspruch 1, **dadurch gekennzeichnet, dass** sie auch für die folgenden Verbindungen gebildet ist:
| **Name des Wirkstoffs** | **% w/v** |
|---|---|
| 2,5-Dihydroxytoluol | 0.00 - 0.16 |
| Nonan | 0.00 - 0.06 |
| Tricyclen | 0.00 - 0.04 |
| 3-Methyleno-1,5,5-trimethylcyclohexen | 0.00 - 0.09 |
| Pseudolimonen | 0.00 - 0.13 |
| (+)-4-Caren | 0.00 - 0.09 |
| 1,3,8-p-Mentatrien | 0.00 - 0.08 |
| Mircenol | 0.00 - 0.17 |
| Bornylchlorid | 0.00 - 0.04 |
| α-Terpineol | 0.00 - 0.13 |
| trans, 4,5-Epoxy-Caran | 0.00 - 0.19 |
| 1-Decanol | 0.00 - 0.09 |
| trans-2-Caren-4-ol | 0.00 - 0.14 |
| (+)-trans-Crisantenil acetat | 0.00 - 0.08 |
| trans-Pinocarveol | 0.00 - 0.11 |
| y-Elemene | 0.00 - 0.09 |
| Myrcenol | 0.00 - 1.10 |
| (-)-β-Elemen | 0.00 - 0.08 |
| α-Bergamoten | 0.00 - 0.15 |
| α-Himachalen | 0.00 - 0.07 |
| (Z)-β-Farnesen | 0.00 - 0.06 |
| α-Muurolen | 0.00 - 0.08 |
| Isoleden | 0.00 - 0.19 |
| α-Longipinen | 0.00 - 0.15 |
| (+)-y-Cadinen | 0.00 - 0.09 |
| Valencen | 0.00 - 0.07 |
| Eudesma-3,7 (11)-dien | 0.00 - 0.21 |
| Cariofilenyl alkohol | 0.00 - 0.09 |
| y-Eudesmol | 0.00 - 0.10 |

3. Nahrungsergänzungsmittel für Geflügel, **dadurch gekennzeichnet, dass** es aus 1 kg ätherischem Öl nach den Ansprüchen 1 und 2 pro Tonne Lebensmittel besteht.

## Revendications

1. Composition de l'huile essentielle de zeste de citron de Citrus aurantifolia utile comme promoteur de croissance chez la volaille.
| | |
|---|---|
| γ-Terpinéol | 0.64-2.48 |
| Caryophyllène | 0.16-0.77 |
| α-Bergamotène | 0.32-1.84 |
| α-Humulène | 0.03-0.22 |
| γ-Sélinène | 0.06-0.28 |
| (-)-β-Cadinène | 0.12-0.57 |
| α-Farmésène | 0.43-1.71 |
| α-Sélinène | 0.04-0.18 |
| β-Bisabolène | 0.62-2.13 |
| β-Maaliène | 0.11-0.61 |

2. Composition de l'huile essentielle de zeste de citron selon la revendication 1, **caractérisée en ce qu'**elle est également constituée pour les composés suivants :
| **Nom du composé** | **% p/v** |
|---|---|
| 2,5-dihydrotoluène | 0.00-0.16 |
| Nonane | 0.00-0.06 |
| Tricyclène | 0.00-0.04 |
| 3-Méthyléno-1,5,5-triméthylcyclohexène | 0.00-0.09 |
| Pseudolimonène | 0.00-0.13 |
| (+)-4-Carène | 0.00-0.09 |
| 1,3,8-p-Menthatriène | 0.00-0.08 |
| Myrcénol | 0.00-0.17 |
| Chlorure de bornyle | 0.00-0.04 |
| α-Terpinéol | 0.00-0.13 |
| Trans, 4,5-époxy-Carane | 0.00-0.19 |
| Décan-1-ol | 0.00-0.09 |
| Trans-2-caren-4-ol | 0.00-0.14 |
| Acétate de (+)-trans-crisanténil | 0.00-0.08 |
| Trans-Pinocarvéol | 0.00-0.11 |
| γ-élémène | 0.00-0.09 |
| Myrcénol | 0.00-1.10 |
| (-)-β-élémène | 0.00-0.08 |
| α-Bergamotène | 0.00-0.15 |
| α-Himachalène | 0.00-0.07 |
| (Z)-β-Farnésène | 0.00-0.06 |
| α-Muurolène | 0.00-0.08 |
| Isolédène | 0.00-0.19 |
| α-Longipinène | 0.00-0.15 |
| (+)-γ-Cadinène | 0.00-0.09 |
| Valencène | 0.00-0.07 |
| Eudesma-3,7(11 )-diène | 0.00-0.21 |
| Cariofilenyl alcool | 0.00-0.09 |
| y-Eudesmol | 0.00-0.10 |

3. Complément alimentaire pour la volaille **caractérisé en ce qu'**il est constitué de 1 kg d'huile essentielle selon les revendications 1 et 2, par tonne métrique d'aliments.
